Europäisches Patentamt

European Patent Office

Office européen des brevets

(19)

(11) Publication number: **0 294 210 B1**

## (12) EUROPEAN PATENT SPECIFICATION

(45) Date of publication of patent specification: **27.10.93**

(51) Int. Cl.5: **A61B 17/06**, B21G 1/08, C22C 38/44, C22C 38/52

(21) Application number: **88305073.4**

(22) Date of filing: **03.06.88**

(54) **Surgical needles from high strength steel alloy and method of producing the same.**

(30) Priority: **05.06.87 US 58713**

(43) Date of publication of application:
**07.12.88 Bulletin 88/49**

(45) Publication of the grant of the patent:
**27.10.93 Bulletin 93/43**

(84) Designated Contracting States:
**DE FR GB IT**

(56) References cited:
EP-A- 0 168 833    EP-A- 0 185 523
CH-A- 450 334    DE-C- 835 137
GB-A- 146 330    GB-A- 2 113 588
US-A- 3 861 909

(73) Proprietor: **ETHICON INC.**
**U.S. Route 22**
**Somerville New Jersey 08876(US)**

(72) Inventor: **Bendel, Lee P.**
**28 Country Hill Road**
**Lebanon N.J. 08837(US)**

(74) Representative: **Jones, Alan John et al**
**CARPMAELS & RANSFORD**
**43 Bloomsbury Square**
**London, WC1A 2RA (GB)**

**Description**

The invention relates to surgical needles made from a high strength steel alloy, and to a method for producing such needles.

Background of the Invention

Surgical needles are used in wound closure to pull the suture through tissue. In order to minimize trauma caused by suturing, the needle should be as small as possible. Therefore, high strength is a desirable property of the material from which the needle is made so that the size of the needle can be kept small. The important mechanical characteristics of the needle are ductility, sharpness, and bending strength. Ductility is important so that the needle will not break during normal use, sharpness so that the needle will penetrate tissue easily with a minimum of trauma, and bending strength so that the needle will resist bending during use. High strength is also important for retaining a sharp point or edge. This invention is based upon the discovery that a certain high strength alloy can be used to fabricate surgical needles which are stronger than any other surgical needles known to the inventor herein.

Brief Summary of the Invention

The surgical needles of the invention are made from an age hardenable iron-base alloy containing, by weight, 9-13 per cent chromium, 8-16 per cent cobalt, 4-8 per cent nickel, x per cent molybdenum and y per cent tungsten, the balance being iron and incidental impurities,
wherein (x + Ay) is between 4 and 8, A being any number from 0.62 to 0.83, and
wherein said elements are balanced to provide an austenite retention index ("ARI") value of from 18 to 22.8, as calculated from the equation:

$$ARI = \% \ Ni + 0.8 \ (\% \ Cr) + 0.6(x + Ay) + 0.3 \ (\% \ Co).$$

The invention also provides a process for making surgical needles from said alloy which comprises cold drawing said alloy to form a wire, shaping the needle from the cold drawn wire, and then age-hardening the shaped needle.

The Prior Art

Caton, In U.S. Patent No.3,861,909 discloses the high strength steel alloy that is used to fabricate the surgical needles of this invention.

EP-A-0185523 (Ethicon, Inc.) discloses a photoetching process for making surgical needles from stainless steel or molybdenum. The specification also describes a previously known method for the manufacture of surgical needles, the method comprising the steps of cold drawing an iron-base alloy, shaping the needles from the cold drawn wire, followed by heat treating the needles to increase their hardness without imparting brittleness. The tempered needles are then polished.

Surgical needles have been made commercially from several types of stainless steel, including S45500 stainless steel, S42000 stainless steel, and S30200 stainless steel. (These materials are usually referred to as "455", "420", and "302" stainless steels, respectively).

Brief Description of the Drawings

Fig. 1 is a perspective view of a surgical needle of the invention;
Fig. 2 is a graph of ultimate tensile strength vs. diameter for wires of various types of steels, including the steel used in this invention.
Figs. 3a and 3b are graphs of the angular deflection, in degrees, vs. bending moment, for two different sizes of needles of the invention and comparably sized needles made of 455 stainless steel; and
Fig. 4 is a graph of ultimate tensile strength vs. length increase for drawn wire used in the invention, both as drawn and drawn plus age hardened.

## Detailed Description of the Invention

The alloy used to produce the surgical needles of the invention is an age hardenable stainless iron based alloy which consists essentially of, in weight per cent:

|  | Broad | Preferred |
|---|---|---|
| Carbon | 0-0.1 | <0.01 |
| Nitrogen | 0-0.1 | <0.01 |
| Manganese | 0-2 | <0.1 |
| Silicon | 0-1 | <0.1 |
| Phosphorus | 0-0.05 | <0.01 |
| Sulfur | 0-0.05 | <0.01 |
| Tungsten | 0-12.8 | 0-9.6 |
| Boron | 0-0.02 | 0.001-0.003 |
| Chromium | 9-13 | 9.5-11.5 |
| Cobalt | 8-16 | 9.5-13.5 |
| Molybdenum | 4-8 | 5-6 |
| Nickel | 4-8 | 5-7 |

The balance is essentially iron, and incidental impurities, and the elements nickel, chromium, molybdenum, tungsten and cobalt are added in such proportions that the austenite retention index is within the range of from about 18 to about 22.8, as calculated from the equation: $ARI = \% \ Ni + 0.8(\% \ Cr) + 0.6(x + Ay) + 0.3(\% \ Co)$ and with the elements carbon, nitrogen, manganese, and silicon being controlled as described below. The relationship among the elements Ni, Cr, Mo, W and Co is based upon the relative effect of those elements in depressing the $M_s$ temperature (the temperature at which austenite starts to transform to martensite upon cooling) of the alloy with the effect of nickel equal to unity. For the stated broad range of the alloy, chromium was determined to be 80% as effective in depressing the temperature ($M_s$) at which the transformation of austenite to martensite begins, molybdenum was found to be 60% as effective as nickel, and cobalt was found to be 30% as effective as nickel.

Tungsten can be substituted for molybdenum in this alloy. At any level of molybdenum, the amount of tungsten required to replace a given amount of molybdenum with an equivalent effect is in the proportion of about 1.2% to 1.6% tungsten to 1% molybdenum.

When carbon or nitrogen are present in solid solution, in the amounts permitted by the table set forth above, the amount of each should be multiplied by thirty (30), and the resulting product(s) should be added in calculating the ARI.

If manganese is present in the alloy in amounts above 0.5%, the manganese present should be taken into account in calculating the ARI by adding one half of all the per cent manganese.

If silicon is present in amounts above about 0.5%, the silicon present should be taken into account when calculating the ARI by multiplying the percentage of silicon by 1.5 and adding the product to the ARI calculation.

The alloy may be produced by conventional procedures by melting the components, preferably by vacuum induction melting. Preferably, a double melting procedure is employed in which the components are first melted in air or in a vacuum induction furnace, and the melt is cast in the form of a consumable electrode. The said electrode is then remelted in vacuum or under a controlled atmosphere. The alloy can be solution treated from about 760°C (1400°F) to 1093°C (2000°F), and preferably from about 816°C (1500°F) to about 982°C (1800° F), for a sufficient time to ensure that the austenizing process is complete. About one hour for each inch of thickness is usually sufficient.

The alloy may be hot worked into wire rod using typical processing conditions for stainless steel materials. Hot rod size should be approximately 1/4 inch in diameter. After the final hot rolling operation the rod should be solution treated at 760°C (1400°F) to 1093°C (2000°F) for about 2 to 4 hours followed by air cooling. The rod should then be acid cleaned.

Wire drawing to needle wire sizes is accomplished by drawing with standard wire drawing dies made of materials such as tungsten carbide or diamond. The amount of reduction is typically 20% to 30% reduction in area per die. After the wire has been reduced in area from about 80 to 90%, it should be annealed, preferably in a protective atmosphere such as hydrogen. Strand annealing is the preferred method to insure uniform properties. (Strand annealing is a process of annealing wire in a tube furnace where the hard wire is pulled through the furnace as a single strand of wire. An inert atmosphere such as hydrogen is maintained in the furnace.) Strand annealing speed and temperature is selected to insure the optimum aging response and work hardening rate. To illustrate, for a 0.89mm (35-mil)wire, one-half minute in a zone heated to 1063°C (1945°F) has been found to be acceptable. Several sequences of drawing through several dies followed by annealing will usually be required. The final drawing is accomplished as above (i.e., drawn through several dies followed by annealing); however, the total reduction in cross-sectional area in the final drawing sequence can be as much as 99%.

Needle making can be accomplished using standard machinery and tooling. The needle making process includes straightening and cutting the wire to an appropriate length, applying a point of the selected geometry, flattening, and curving the needle to the selected dimensions. The suture end of the needle is channeled or drilled or otherwise modified to permit suture attachment. The needle is then polished to a smooth surface finish.

Age hardening is performed to develop the full strength potential of the material. Age hardening is preferably accomplished in the absence of air. Vacuum, hydrogen, or inert gasses are used to protect the surface during the age hardening (heating) operation. Illustrative age hardening conditions are to heat the needle to about 482°C (900°F) to 593°C (1100°F), preferably to about 510°C (950°F) to 538°C (1000°F), for about 2 to 4 hours, followed by cooling in air.

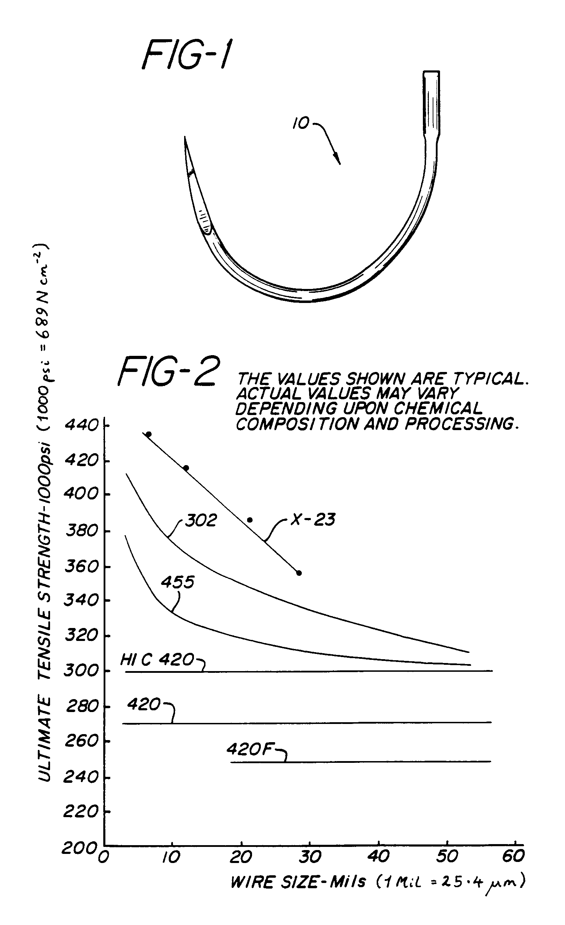

The strength values shown in Figure 2 were obtained from the following sources: The 455, 420, 420F (S42020 stainless steel), and High Carbon 420 stainless steel alloy data are from tests conducted on various lots of wire used in the normal production of commercial surgical needles made from these stainless steels. The 302 stainless steel alloy data are from tests conducted on wire samples purchased from various suppliers, as well as wire drawn by or on on behalf of the inventor herein. X-23 is an alloy of the invention whose composition is set forth below. The X-23 data are from tests conducted by the inventor herein on wire samples drawn, shaped into needles, and then age hardened.

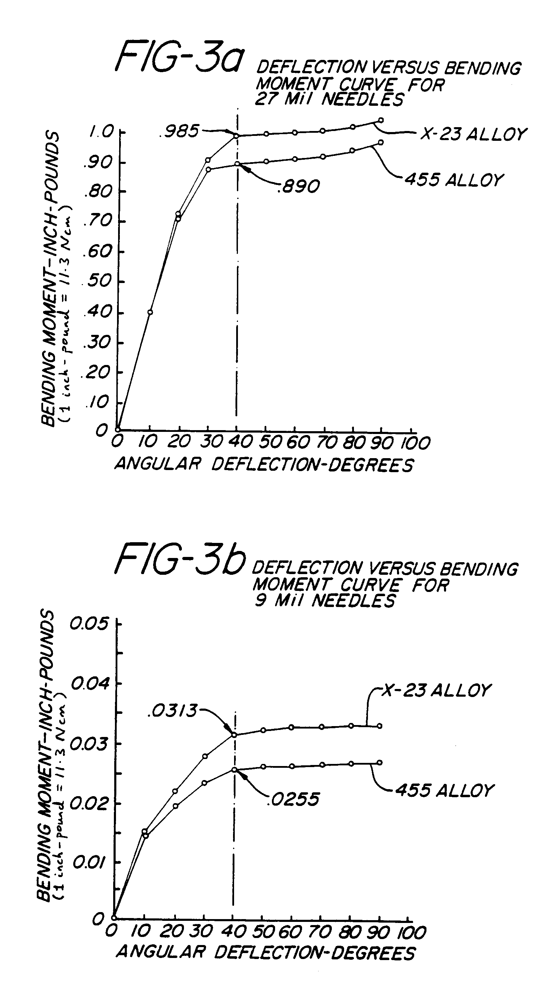

Figures 3a/3b. The X-23 needle test results shown in these Figures were obtained from needles that were produced using the method described above. The test results on the 455 needles were obtained from standard production lots of needles made of 455 stainless steel. The data were obtained on a bend tester to determine the effect of needle strength on the resistance to bending. In the bend test, jaws grip the needle at the location where a surgeon would normally grip it. The portion of the needle projecting out from the gripping mechanism is pressed against a knife edge attached to a load cell. The gripping mechanism is rotated. The force on the load cell is measured as a function of angular rotation up to 90°. The force times the moment arm (i.e., distance from the gripping mechanism to the knife edge) is the bending moment. The needle is removed from the gripping mechanism and is reshaped by hand to evaluate ductility. Needles which can be bent as described above and reshaped without breaking are considered to have good ductility. Surgical needles made of 420 and 420F stainless steel can typically survive one bending and reshaping cycle without breaking. Surgical needles made of 455 stainless steel and X-23 alloy can typically survive two to three bending and reshaping cycles without breaking.

The needles of the invention have a bending moment in the test described above, when bent to 40°, of at least about 10.6Ncm (0.94 inch-pound) in the 686μm (27 mil) size and at least about 0.33Ncm (0.029 inch-pound)in the 229μm (9 mil) size.

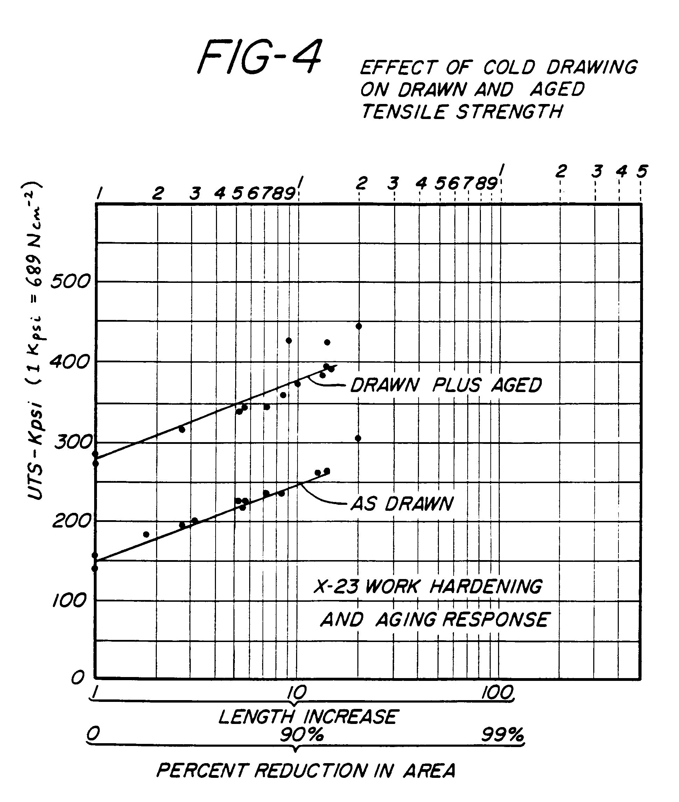

Figure 4. The X-23 wires whose test results are shown in this figure were prepared using the wire drawing and age hardening processes described above.

X-23 is an alloy having the following analysis:

| Element | Weight per cent |
|---------|-----------------|
| Cr | 9.89 |
| Co | 15.12 |
| Mo | 6.02 |
| Ni | 5.0 |
| C | 0.001 |
| Mn | <0.01 |
| Si | 0.01 |
| P | <0.005 |
| S | 0.003 |
| B | 0.0022 |
| N | 0.003 |
| Fe | Balance |

Figure 1 shows a typical surgical needle 10 made from the alloy of the invention.

## Claims

1. A surgical needle having high strength and good ductility, which surgical needle comprises a cold drawn, age hardened iron-base alloy containing, by weight, 9-13 per cent chromium, 8-16 per cent cobalt, 4-8 per cent nickel, x per cent molybdenum and y per cent tungsten, the balance being iron and incidental impurities,

wherein (x + Ay) is between 4 and 8, A being any number from 0.62 to 0.83, and

wherein said elements are balanced to provide an austenite retention index ("ARI") value of from 18 to 22.8, as calculated from the equation:

$$ARI = \%\ Ni + 0.8(\%\ Cr) + 0.6(x + Ay) + 0.3(\%\ Co).$$

2. A surgical needle according to claim 1 wherein said alloy contains no tungsten other than tungsten present as an incidental impurity.

3. A surgical needle according to claim 2 wherein said alloy contains 9.5-11.5 weight per cent chromium, 9.5-13.5 weight per cent cobalt, 5-6 weight per cent molybdenum, and 5-7 weight per cent nickel.

4. A process for producing a surgical needle having high strength and good ductility, which process comprises:

cold drawing an iron-base alloy containing, by weight, 9-13 per cent chromium, 8-16 per cent cobalt, 4-8 per cent nickel, x per cent molybdenum and y per cent tungsten, the balance being iron and incidental impurities, in which (x + Ay) is between 4 and 8, A being any number between 0.62 and 0.83, and in which said elements are balanced to provide an austenite retention index (ARI) value of from 18 to 22.8, as calculated from the equation:

$$ARI = \%\ Ni + 0.8(\%\ Cr) + 0.6(x + Ay) + 0.3(\%\ Co);$$

shaping said needle from the cold drawn wire; and
age hardening the shaped needle.

5. A process for producing a surgical needle according to claim 4 wherein said alloy contains no tungsten other than tungsten present as an incidental impurity.

6. A process for producing a surgical needle according to claim 5 wherein said alloy contains 9.5-11.5 weight per cent chromium, 9.5-13.5 weight per cent cobalt, 5-6 weight per cent molybdenum, and 5-7 weight per cent nickel.

## Patentansprüche

1. Chirurgische Nadel mit hoher Festigkeit und guter Duktilität, die eine kaltgezogene, getemperte Legierung auf Eisenbasis umfaßt, die bezogen auf das Gewicht 9 bis 13 % Chrom, 8 bis 16 % Cobalt, 4 bis 8 % Nickel, x % Molybdän und y % Wolfram und als Rost Eisen sowie zufällig auftretende Verunreinigungen enthält,

wobei (x + Ay) zwischen 4 und 8 liegt, A eine beliebige Zahl Von 0,62 bis 0,83 ist, und

worin diese Elemente derart abgeglichen sind, daß sie einen Austenit-Retentionsindex ("ARI") mit einem Wert von 18 bis 22,8 ergeben, Wenn er mittels der Gleichung:

$$ARI = \%\ Ni + 0,8\ (\%\ Cr) + 0,6\ (x + Ay) + 0,3\ (\%\ Co)$$

berechnet wird.

2. Chirurgische Nadel nach Anspruch 1, worin die Legierung kein Wolfram enthält, außer demjenigen Wolfram, das als Zufällige Verunreinigung vorliegt.

5

**3.** Chirurgische Nadel nach Anspruch 2, worin die Legierung 9,5 bis 11,5 Gew.-% Chrom, 9,5 bis 13,5 Gew.-% Cobalt, 5 bis 6 Gew.-% Molybdän und 5 bis 7 Gew.-% Nickel enthält.

**4.** Verfahren zur Herstellung einer chirurgischen Nadel mit hoher Festigkeit und guter Duktilität, umfassend:

das Kaltziehen einer Legierung auf Eisenbasis, die, bezogen auf das Gewicht, 9 bis 13 % Chrom, 8 bis 16 % Cobalt, 4 bis 8 % Nickel, x % Molybdän und y % Wolfram enthält, wobei der Rest Eisen und zufällige Verunreinigungen sind, und wobei (x + Ay) zwischen 4 und 8 liegt, A eine beliebige Zahl zwischen 0,62 und 0,83 ist, und wobei diese Elemente derart abgeglichen sind, daß sie einen Austenit-Retentionsindex (ARI) mit einem Wert von 18 bis 22,8 ergeben, wenn er mittels der Gleichung:

$$ARI = \% \ Ni + 0,8 \ (\% \ Cr) + 0,6 \ (x + Ay) + 0,3 \ (\% \ Co)$$

berechnet wird,

das Formen der Nadel aus einem kaltgezogenen Draht und
das Tempern der gezogenen Nadel.

**5.** Verfahren zur Herstellung einer chirurgischen Nadel nach Anspruch 4, bei dem die Legierung kein Wolfram enthält, außer demjenigen Wolfram, das als zufällige Verunreinigung vorliegt.

**6.** Verfahren zur Herstellung einer chirurgischen Nadel nach Anspruch 5, bei dem die Legierung 9,5 bis 11,5 Gew.-% Chrom, 9,5 bis 13,5 Gew.-% Cobalt, 5 bis 6 Gew.-% Molybdän und 5 bis 7 Gew.-% Nickel enthält.

**Revendications**

**1.** Aiguille chirurgicale ayant une résistance élevée et une bonne ductilité, laquelle aiguille chirurgicale se compose d'un alliage à base de fer durci par vieillissement et étiré à froid contenant, en poids 9 à 13 % de chrome, 8 à 16 % de cobalt, 4 à 8 % de nickel, x % de molybdène, et y % de tungstène, le reste étant du fer et des impuretés accidentelles,

dans laquelle (x + Ay) est compris entre 4 et 8, A étant un nombre quelconque compris entre 0,62 et 0,83, et

dans laquelle lesdits éléments sont équilibrés pour fournir une valeur de l'indice de rétention austénite (ARI) compris entre 18 et 22,8, calculé à partir de l'équation :

$$ARI = \% \ Ni + 0,8 \ (\% \ Cr) + 0,6(x + Ay) + 0,3 \ (\% \ Co)$$

**2.** Aiguille chirurgicale selon la revendication 1 dans laquelle ledit alliage ne contient pas de tungstène autre que le tungstène présent dans les impuretés accidentelles.

**3.** Aiguille chirurgicale selon la revendication 2 dans laquelle ledit alliage contient en poids 9,5 à 11,5 % de chrome, 9,5 à 13,5 % de cobalt, 5 à 6 % de molybdène, et 5 à 7 % de nickel.

**4.** Procédé pour fabriquer une aiguille chirurgicale ayant une résistance élevée et une bonne ductilité, lequel procédé comprend:

l'étirement à froid d'un alliage à base de fer durci par vieillissement et tiré à froid contenant, en poids, 9 à 13 % de chrome, 8 à 16 % de cobalt, 4 à 8 % de nickel, x % de molybdène, et y % de tungstène, le reste étant du fer et des impuretés accidentelles, dans lequel (x + Ay) est compris entre 4 et 8, A étant un nombre quelconque compris entre 0,62 et 0,83, et dans lequel lesdits éléments sont équilibrés pour fournir une valeur de l'indice de rétention austénite (ARI) compris entre 18 et 22,8, calculé à partir de l'équation :

$$ARI = \% \ Ni + 0,8 \ (\% \ Cr) + 0,6 \ (x + Ay) + 0,3 \ (\% \ Co);$$

le façonnage de ladite aiguille à partir du fil étiré à froid; et
le durcissement par vieillissement de l'aiguille façonnée.

5. Procédé pour fabriquer une aiguille chirurgicale selon la revendication 4 dans lequel ledit alliage ne contient pas de tungstène autre que le tungstène présent dans les impuretés accidentelles.

6. Procédé pour fabriquer une aiguille chirurgicale selon la revendication 5 dans lequel ledit alliage contient en poids, 9,5 à 11,5 % de chrome, 9,5 à 13,5 % de cobalt, 5 à 6 % de molybdène, et 5 à 7 % de nickel.

# FIG-1

10

# FIG-2

THE VALUES SHOWN ARE TYPICAL.
ACTUAL VALUES MAY VARY
DEPENDING UPON CHEMICAL
COMPOSITION AND PROCESSING.

ULTIMATE TENSILE STRENGTH-1000psi ($1000_{psi} = 689 \, N \, cm^{-2}$)

302

X-23

455

HI C 420

420

420F

WIRE SIZE-Mils ($1 \, Mil = 25.4 \, \mu m$)

FIG-3a DEFLECTION VERSUS BENDING MOMENT CURVE FOR 27 Mil NEEDLES

FIG-3b DEFLECTION VERSUS BENDING MOMENT CURVE FOR 9 Mil NEEDLES

9

FIG-4

EFFECT OF COLD DRAWING ON DRAWN AND AGED TENSILE STRENGTH